# EUROPÄISCHE PATENTSCHRIFT

(11) **EP 2 482 079 B1**
(45) Veröffentlichungstag und Bekanntmachung des Hinweises auf die Patenterteilung: **06.01.2021**
(21) Anmeldenummer: 12000411.4
(22) Anmeldetag: 24.01.2012
(51) Int. Cl.: G01N 35/02, G01N 35/00, C12M 1/36, C12M 1/22, C12M 1/00, G01N 35/04

(54) **Vorrichtung zur Lagerrung und Handhabung von Petrischalen, Lagervorrichtung und Lagerschacht für Laborobjekte**
Device for storing and handling Petri dishes, storage device and storage shaft for laboratory objects
Dispositif de stockage et de manipulation de boîtes de Pétri, dispositif de stockage et puits de stockage pour objets de laboratoire

(30) Priorität: 28.01.2011 CH 1512011; 06.05.2011 CH 7832011
(43) Veröffentlichungstag der Anmeldung: 01.08.2012
(73) Patentinhaber: LICONIC AG, 9493 Mauren (LI)
(72) Erfinder: Malin, Cosmas, 9493 Mauren (LI)
(74) Vertreter: Sutter, Kurt

(56) Entgegenhaltungen:
- US-A- 3 844 896
- US-A1- 2003 031 602
- US-A1- 2003 044 321
- US-A1- 2004 115 101
- US-A1- 2006 289 371
- US-A1- 2007 172 396

## Beschreibung

### Gebiet der Erfindung

Die Erfindung betrifft eine Vorrichtung zur Lagerung und Handhabung von Laborobjekten, insbesondere Petrischalen. Weiter betrifft die Erfindung eine Laborvorrichtung.

### Hintergrund

Petrischalen sind flache, runde, durchsichtige Schalen mit übergreifendem Deckel, die in der Biologie, Medizin oder Chemie verbreitet zum Einsatz kommen. Dabei werden Petrischalen zur Kultivierung von Mikroorganismen und Zellkulturen genutzt. Eine flache Schicht aus einem gelförmigen Nährmedium ist in die Petrischale gefüllt und versorgt die wachsenden Mikroorganismen mit Wasser und den notwendigen Nährstoffen.

Anwendungsspezifische Mikroorganismen werden lokal in das Nährmedium eingebracht. Anschliessend werden die Petrischalen meist mit dem Deckel nach unten und dem Nährboden nach oben inkubiert. Bei dieser Lagerung lastet das Gewischt der Platte auf dem Deckel, wodurch der Verschluss zwischen Deckel und Schale verbessert wird. Überschüssiges Wasser bildet sich nicht auf dem Nährboden, sondern sammelt bevorzugt sich auf dem Deckel.

Während der Inkubationszeit wird das Wachstum der Kulturen mehrmals visuell inspiziert. In Anwendungen in denen mit grossen Zahlen von Platten gearbeitet wird, besteht Bedarf für eine Automatisierung des Vorgangs. Dabei wird nach bestimmten zeitlichen Vorgaben jeweils eine Petrischale dem Inkubator bzw. der Lagervorrichtung entnommen und einer Inspektionsvorrichtung zugeführt. Für die Inspektion sollen der Inspektionsvorrichtung die Platten mit dem Nährboden nach unten zugeführt werden.

Zum Transferieren der Petrischalen zwischen Lagervorrichtung und Inspektionsvorrichtung wird eine Transfervorrichtung benötigt. Üblicherweise besitzt diese einen Schwenkarm, der an einer Wandung der Inkubationskammer bzw. Lagervorrichtung angeordnet ist. Eine Petrischale wird von einem Vakuumsauger, der am äusseren Ende des Schwenkarms angeordnet ist, erfasst und mittels einer Drehbewegung von 180 Grad aus dem Inkubator entnommen und gleichzeitig gedreht. Bei der Inspektionsvorrichtung muss der Vakuumsauger für die Inspektion entfernt werden.

Weiter sind zur Lagerung von Laborobjekten Lagerschächte bekannt, welche jeweils Platz für mehrere Laborobjekte übereinander bieten.

US 2003/0044321 beschreibt eine Vorrichtung, mit welcher Schalen mit Deckel manipuliert werden können. Sie besitzt einen Greifer, um den Boden der Schalen zu halten, sowie eine Anhebevorrichtung für den Deckel.

### Darstellung der Erfindung

Es ist die Aufgabe zu lösen, eine Vorrichtung zur Lagerung und Handhabung von Petrischalen bereitzustellen, welche eine effiziente Handhabung der Petrischalen erlaubt.

Diese Aufgabe wird von der Vorrichtung gemäss Anspruch 1 gelöst. Demgemäss weist die Transfervorrichtung einen Greifer auf, mit welchem jeweils eine Petrischale ohne den Deckel an den Seitenwänden des Bodens lateral ergreifbar ist. Der Vorteil dieser Lösung besteht darin, dass der Greifer die Petrischale im Gegensatz zu einem Vakuumsauger im Wesentlichen nur seitlich, nicht aber mittig beaufschlägt, so dass die Petrischale für die Inspektion einfacher zugänglich bleibt.

Weiter besitzt die Transfervorrichtung eine Übergabestation zur Aufnahme einer Petrischale mit dem Deckel nach unten. Von dieser wird jeweils eine Petrischale ohne Deckel vom Greifer ergriffen und nach oben angehoben, so dass der Deckel auf der Übergabestation verbleibt.

Weiter ist vorgesehen, dass die Transfervorrichtung einen Wendeantrieb aufweist, der so ausgestaltet ist, dass der Greifer mit der aufgenommenen Petrischale vertikal über dem Deckel gewendet wird. Es zeigt sich, dass so die Kontaminationsgefahr reduziert werden kann, indem Tropfen, die sich beim Wenden der Petrischale z.B. aufgrund des Luftzugs lösen, nach unten in den Deckel fallen und nicht unkontrolliert an anderen Stellen in der Vorrichtung deponiert werden.

In einer weiteren vorteilhaften Ausführung besitzt die Vorrichtung einen Liftantrieb, mit welchem der Greifer über der Übergabestation und relativ zu dieser vertikal verschaben werden kann, d.h. in einer vertikalen Translationsbewegung bewegt werden kann. Hierzu kann entweder der Greifer, oder die Übergabestation, oder beide Teile bewegt werden. Dank dem vertikalen Anheben kann eine Verkantung des vom Greifer aufgenommenen Bodens der Petrischale in deren Deckel vermieden werden.

In der Regel wird die Lagervorrichtung eine Vielzahl von Lagerplätzen für Petrischalen bilden, und die Vorrichtung wird so ausgestaltet sein, dass die Petrischalen in den Lagerplätzen mit dem Deckel nach unten gelagert sind. Zudem kann zusätzlich zur Transfervorrichtung eine Umschlagvorrichtung vorgesehen sein, um die Petrischalen zwischen den Lagerplätzen und der Transfervorrichtung hin- und her zu transportieren. Die Umschlagvorrichtung kann mindestens teilweise in der Lagervorrichtung angeordnet sein.

Die Lagerplätze für die Petrischalen werden vorteilhaft von mehreren Lagerschächten gebildet. Jeder dieser Lagerschächte weist mehrere Lagerplätze übereinander auf, so dass die Petrischalen wie in einem Turm gelagert werden können. In einer besonders vorteilhaften Ausführung werden diese Lagerschächte auf einem Karussell angeordnet, so dass jeder davon durch Drehen des Karussells zur Umschlagvorrichtung gebracht werden kann.

Die Lagervorrichtung soll vorzugsweise eine einfache Montage und Demontage der Lagerschächte erlauben. Gemäss einem abhängigen Anspruch besitzt die Lagervorrichtung mindestens einen Lagerschacht, wobei jeder Lagerschacht mehrere Lagerplätze zur Aufnahme jeweils eines Laborobjekts übereinander aufweist. Am Boden jedes Lagerschachts ist mindestens eine Öffnung angeordnet, in welche mindestens ein Stift einer Unterlage des Lagerschachts eingreift. Von oben sind die Lagerschächte mittels einer federnden Zunge gehalten. Durch Deformation der Zunge kann ein Lagerschacht angehoben, von den Stiften gelöst und der Lagervorrichtung entnommen werden.

Weiter besitzt die Lagervorrichtung vorzugsweise einen Lagerschacht zur Aufnahme der Petrischalen. Der Lagerschacht besitzt eine Rückwand und zwei Seitenwände. Zumindest an den Seitenwänden, optional auch an der Rückwand, sind Auflagen für die periphere Aufnahme der Petrischalen vorgesehen. Die Rückwand geht über zwei Übergangsbereiche in die beiden Seitenwände über. Jeder Übergangsbereich ist gerundet, oder er verläuft schräg zur Rückwand und zur jeweiligen angrenzenden Seitenwand. Diese Ausgestaltung, welche die runde Bauform von Petrischalen berücksichtigt, hat den Vorteil, dass die Lagerschächte eng in einem Kreis, beispielsweise auf einem Karussell angeordnet werden können, so dass eine Lagerung mit hoher räumlicher Kapazität möglich ist.

In einem weiteren Aspekt betrifft die Erfindung eine Laborvorrichtung, welche zum Zusammenwirken mit der Vorrichtung ausgestaltet und vorgesehen ist.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 eine Ansicht einer Anlage von oben,
Fig. 2 eine Ansicht der Transfervorrichtung und der angrenzenden Komponenten,
Fig. 3 einen Teil der Transfervorrichtung,
Fig. 4 eine Ausführung eines Greifers,
Fig. 5 eine von mehreren Auflagen der Übergabestation,
Fig. 6 einen Lagerschacht für Petrischalen,
Fig. 7 ein Detail (Kreis A) des Lagerschachts von Fig. 6,
Fig. 8 eine Befestigungsvariante für die Lagerschächte,
Fig. 9 eine Transfervorrichtung mit zwei Übergabestationen übereinander;
Fig. 10 eine alternative Ausführung zum Greifer nach Fig. 4;
Figur 11 eine Darstellung einer weiteren Transfervorrichtung mit zwei Übergabestationen zur Erläuterung weiterer bevorzugter Ausführungen;
Figur 12 eine weitere Ausführung eines Greifers in schaubildlicher Ansicht;
Figur 13 den Greifer von Figur 12 in Draufsicht von oben;
Figur 14 eine Ansicht einer bevorzugten Variante der Vorrichtung und eines Teils einer damit zusammenwirkenden Laborvorrichtung;
Figur 15 eine schaubildliche Ansicht einer Hebeeinrichtung; und
Figur 16 eine weitere Ausführung der Lagervorrichtung.

### Wege zur Ausführung der Erfindung

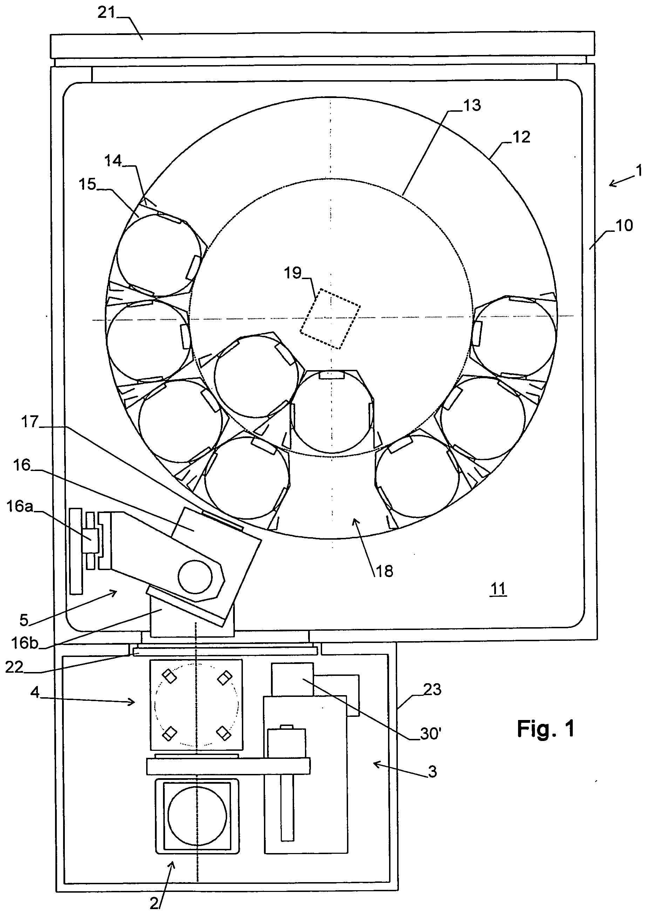

Fig. 1 zeigt eine Vorrichtung zur Lagerung und Handhabung von Petrischalen von oben. Sie umfasst eine Lagervorrichtung 1, eine Inspektionsvorrichtung 2, eine Transfervorrichtung 3, eine Übergabestation 4 sowie eine Umschlagvorrichtung 5. Der Aufbau und die Funktion dieser Komponenten werden im Folgenden im Detail beschrieben.

Die Lagervorrichtung 1 ist vorzugsweise als Inkubator, d.h. als Klimaschrank, ausgestaltet, in dessen Inneren mittels einer geeigneten Klimatisierung eine gewünschte Temperatur und Atmosphäre aufrecht erhalten werden kann. Sie besitzt eine Wand 10, welche einen Innenraum 11 umschliesst. Im Innenraum 11 sind konzentrisch zwei unabhängig voneinander drehbare Karusselle 12 und 13 angeordnet, welche jeweils einen Kreis von Lagerschächten 14 tragen, von denen in Fig. 1 nur ein Teil dargestellt ist. Jeder Lagerschacht 14 bildet eine Vielzahl von Lagerplätzen für Petrischalen 15 übereinander.

In einer Ecke des Innenraums 11 ist weiter die Umschlagvorrichtung 5 angeordnet, die dazu dient, die Petrischalen 15 zwischen den Lagerplätzen und der Transfervorrichtung 3 hin- und her zu transportieren. Hierzu besitzt die Umschlagvorrichtung mindestens einen an einer Liftsäule 16a vertikal verfahrbaren Wagen 16, an welchem eine horizontal ausfahrbare und um eine vertikale Achse schwenkbare Schaufel 17 angeordnet ist. Diese Schaufel kann in der in Fig. 1 gezeigten Schwenkstellung in die Lagerschächte 14 eingefahren werden, um jeweils eine Petrischale 15 aufzunehmen oder abzulegen. Durch eine Lücke 18 im äusseren Kreis der Lagerschächte 14 kann die Schaufel auch auf die Lagerplätze der Lagerschächte des inneren Kreises zugreifen. Hierzu wird Karussell 12 mit dem äusseren Kreis der Lagerschächte 14 so gedreht, dass die Lücke 18 im Bereich der Schaufel 17 zu liegen kommt. Optional kann in der Mitte des inneren Karussells 13 noch eine Hilfseinrichtung 19 vorgesehen sein, welche die Arbeit der Schaufel 17 unterstützt und ihr hilft, die Lagerplätze des inneren Kreises zu beschicken.

Wie in Fig. 1 weiter angedeutet, sind an der Liftsäule 16a in der gezeigten Ausführung mindestens zwei Wagen 16, 16b übereinander angeordnet. In Fig. 1 ist dabei der untere Wagen 16b momentan zur Übergabestation 4 hin gedreht. Durch Verwendung mehrer Wagen 16, 16b, die unabhängig voneinander vertikal verfahrbar sind, kann die Umschlagkapazität erhöht werden. Der untere Wagen 16b bedient die untere Hälfte der Lagervorrichtung 1, und der obere Wagen 16 die obere Hälfte.

Wie weiter ersichtlich, besitzt die Lagervorrichtung 1 in der dargstellten Ausführung eine Benutzertüre 21 sowie eine automatisch öffen- und schliessbare Hilfstüre 22. Um mit der Umschlagvorrichtung 5 eine Petrischale vom Innenraum 11 nach aussen oder in umgekehrter Richtung zu transportieren, wird die Hilfstüre 22 geöffnet.

Ausserhalb der Lagervorrichtung 1 kann z.B. ein Tisch 23 angeordnet sein, der die im Folgenden beschriebenen Komponenten trägt. Der Tisch 23 kann an der Aussenseite der Lagervorrichtung 1 befestigt sein.

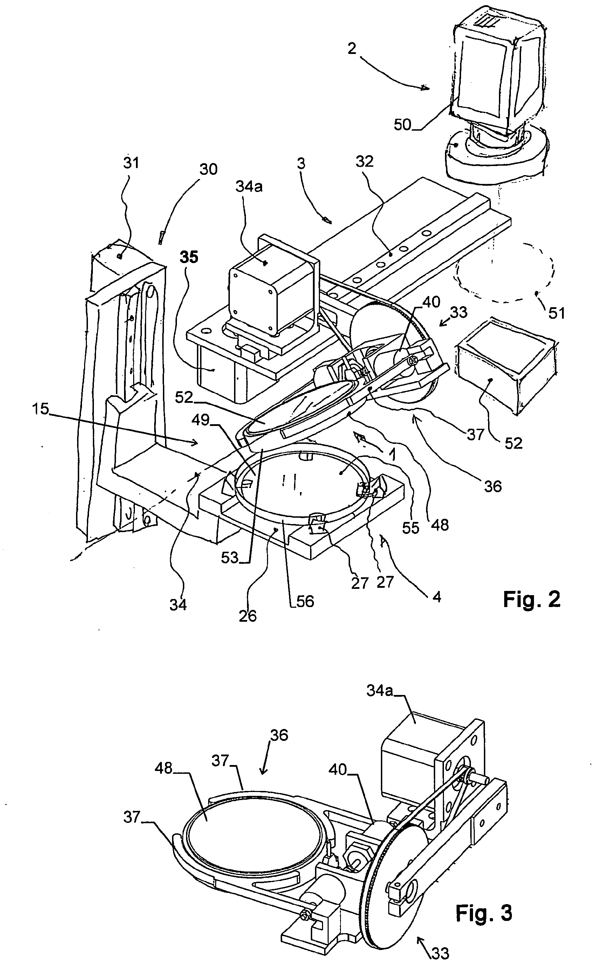

Die Übergabestation 4, welche auch in Fig. 2 dargestellt ist, dient dazu, mindestens eine von der Umschlagvorrichtung 5 ausgetragene Petrischale zu übernehmen, oder temporär mindestens eine Petrischale aufzunehmen, welche sodann von der Umschlagvorrichtung 5 angehoben und in einen der Lagerplätze der Lagervorrichtung 1 abgelegt wird.

Wie in Fig. 2 dargestellt, umfasst die Übergabestation 4 einen Transfertisch 26, auf welchem vier gegenüber dem Transfertisch 26 erhöhte Auflagen 27 angeordnet sind, auf denen eine in der Übergabestation 4 abgelegte Petrischale ruht.

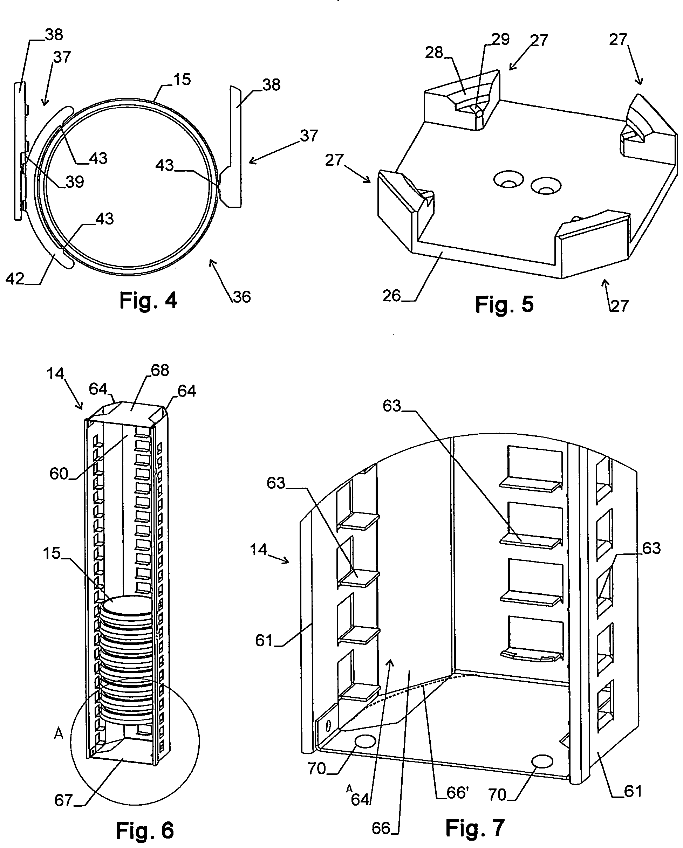

Fig. 5 zeigt den Transfertisch 26 mit vier Auflagen 27 im Detail. Wie ersichtlich, bildet jede Auflage 27 eine seitliche, schräg verlaufende Auflauffläche 28, an welcher die Petrischale zentriert wird. An die Auflauffläche 28 schliesst ein Auflagepolster 29 an, auf dem die Petrischale ruht. Das Auflagepolster 29 ist vorzugsweise aus einem Material mit hoher Haftreibung (d.h. mit höherer Haftreibung als das restliche Material der Auflage 27), so z.B. aus Gummi, so dass die darauf abgelegte Petrischale (bzw. deren Deckel) bei den unten beschriebenen Manipulationen sich möglichst nicht verschiebt.

In der Ausführung nach Fig. 2 ist die Übergabestation 4 vertikal verfahrbar an einem Lift 30 mit Antrieb 31 angeordnet. Demgegenüber ist die Übergabestation 4 in der Ausführung nach Fig. 1 nicht höhenverstellbar - vielmehr ist die Transfervorrichtung 3 an einem Lift 30' höhenverstellbar angeordnet. Beide Varianten sind möglich. Wichtig ist, dass die Transfervorrichtung 3 (bzw. deren im Folgenden beschriebene Greifer) vertikal gegenüber der Übergabestation 4 bewegt werden kann.

Die Transfervorrichtung 3 besitzt eine Schiene 32, entlang der ein Kopf 33 entlang einer horizontalen Achse 34 verfahren werden kann. Hierzu ist ein Horizontalantrieb 35 vorgesehen.

Der Kopf 33, dessen Aufbau aus Fig. 2 und 3 ersichtlich ist, ist um eine Achse parallel zur Achse 34 schwenkbar, wozu ein Wendeantrieb 34a vorgesehen ist, und trägt einen Greifer 36 mit zwei Fingern 37. Der Greifer 36 ist somit um eine horizontale Achse schwenkbar und vertikal gegenüber der Übergabestation 4 beweglich.

Der Greifer 36 besitzt einen Fingerantrieb 40, mit welchem die beiden Finger 37 gegeneinander bewegt werden können, z.B. in einer linearen Bewegung.

Fig. 4 zeigt einen möglichen Aufbau eines Greifers 36 im Detail. Jeder Finger 37 besitzt einen Schaft 38, wobei die beiden Schafte 38 gegeneinander bewegt werden können. An den Fingern 37 sind mindestens drei gegeneinander gerichtete Erhöhungen 43 vorgesehen, um die Seitenwände der Petrischale 15 zu erfassen. Diese dienen dazu, die Wand der Petrischale 15 an definierten Punkten (bzw. entlang definierten Mantellinien) zu beaufschlagen, so dass die Petrischale 15 sicher und ohne Spiel aufgenommen werden kann.

Vorzugsweise ist mindestens eine der Erhöhungen 43 federnd am jeweiligen Finger 37 befestigt. In der Ausführung nach Fig. 4 ist hierzu an einem der Finger 37 eine als Blattfeder ausgestaltete Federung 39 angeordnet, welche ihrerseits ein gebogenes Basisteil 42 hält. Am Basisteil 42 ist mindestens eine der Erhöhungen 43 angeordnet. Im gezeigten Beispiel sind zwei Erhöhungen 43 am Basisteil 42 angeordnet.

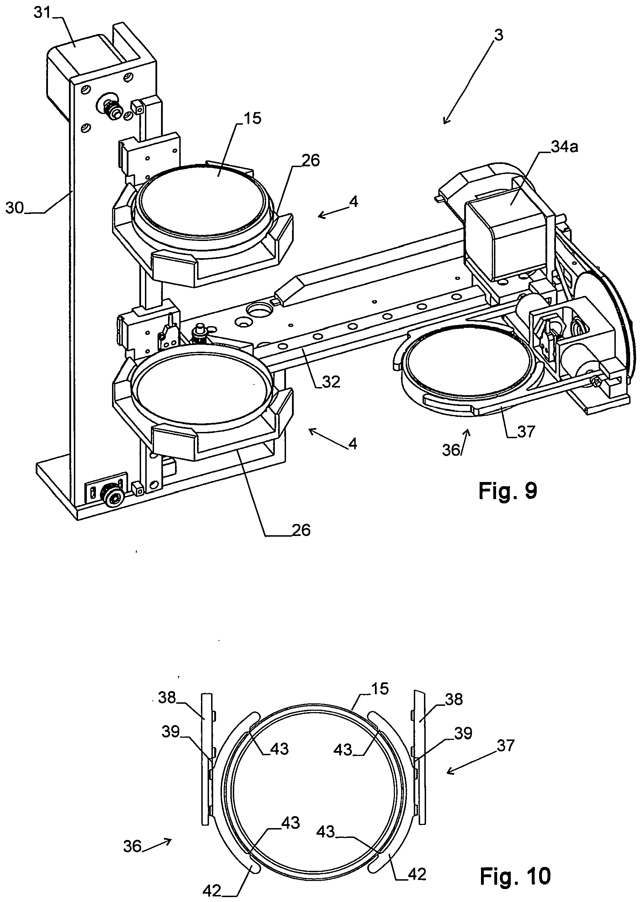

Fig. 10 zeigt eine alternative Ausführung des Greifers 36, bei welcher beide Finger 37 je eine Federung 39 aufweisen, die jeweils einen Basisteil 42 trägt. In diesem Falle ist jeder Basisteil 42 mit jeweils zwei Erhöhungen 43 ausgestattet, da die Federungen 39 ein gewisses Verkippen der Basisteile 42 zulassen und somit auch vier Erhöhungen 43 spielfrei gegen eine nicht ganz runde Petrischale 15 gedrückt werden können.

Um die Funktion der vorliegenden Vorrichtung zu verstehen, ist zunächst der Aufbau der Petrischalen 15 zu erläutern. Eine solche Petrischale ist in Fig. 2 sichtbar. Sie besitzt einen Boden 48 und einen Deckel 49. Wie eingangs erwähnt, befindet sich die zu untersuchende Probe am Boden 48, z.B. in einem am Boden 48 klebenden Nährsubstrat, aber die Petrischale wird in der Lagervorrichtung so gelagert, dass der Boden 48 oberhalb des Deckels 49 ist, d.h. auf dem Deckel 49 aufliegt. Zur Analyse der Petrischale in der im Folgenden noch genauer beschriebenen Inspektionsvorrichtung muss der Boden 48 vom Deckel 49 getrennt und um 180° um eine horizontale Achse geschwenkt werden.

Der Boden 48 besitzt eine runde Bodenfläche 52, an deren Umfang eine periphere, ungefähr zylindrische Bodenwand 53 vorgesehen ist. Der Deckel 49 besitzt seinerseits ebenfalls eine runde Deckelfläche 55, an deren Umfang eine zylindrische Deckelwand 56 vorgesehen ist. Der Innendurchmesser der Deckelwand 56 ist etwas grösser als der Aussendurchmesser der Bodenwand 53. Sind Boden 48 und Deckel 49 zusammengefügt, so greift die Bodenwand 53 in die Deckelwand 56 ein und ruht gegen die Deckelfläche 55.

Wie erwähnt, sind die Petrischalen 15 in den Lagerplätzen der Lagervorrichtung 1 mit dem Deckel 49 nach unten gelagert. Die Umschlagvorrichtung transferiert die Petrischalen 15 ohne sie zu wenden zwischen den Lagerplätzen und der Übergabestation 4. Somit kommen die Petrischalen mit dem Deckel 49 nach unten in der Übergabestation 4 zu liegen. Nun wird der Greifer 36 von oben über den Boden 48 der Petrischale 15 gelegt. Die Finger 37 werden aufeinander zugefahren, so dass sie den Boden 48 der Petrischale 15 an der Bodenwand 53 ergreifen. Jetzt kann der Greifer 36 relativ zur Übergabestation 4 angehoben werden, so dass er den Boden 48 aus dem Deckel 49 zieht. Falls sich zu diesem Zeitpunkt ein Tropfen vom Boden 48 löst, so fällt er in den Deckel.

Nun wird der Greifer 36 mit der aufgenommenen Petrischale gewendet. Dies geschieht vertikal oberhalb des in der Übergabestation 4 liegenden Deckels 49, so dass auch in dieser Phase allfällige sich vom Boden 48 lösende Tropfen in den Deckel 49 fallen. Vorzugsweise wird der Boden 48 nicht von oberhalb des Deckels 49 weg bewegt, bis er um mehr als 90° gewendet wurde.

Nun kann der Greifer 36 mit dem aufgenommenen und gewendeten Deckel 48 mit Hilfe des Horizontalantriebs 35 zur Inspektionsvorrichtung 2 verfahren werden. In der vorliegenden Ausführung handelt es sich um eine optische Inspektionsvorrichtung mit einer Kamera 50, welche ein Bild einer in einer Messposition 51 angeordneten Petrischale zu liefern vermag. Die Messposition 51 befindet sich zwischen der Kamera 50 und einer Lichtquelle 52, um Aufnahmen im Durchlicht zu erzeugen.

Mit dem Greifer 36 kann der aufgenommene und gewendete Deckel 48 zur Messposition 51 verfahren werden. Hierzu wird der Horizontalantrieb 35 verwendet, mit welchem der Kopf 33 entlang der Schiene 32 bewegt werden kann. Dabei ist die Vorrichtung so ausgestaltet, dass der Deckel 48 der Petrischale während der Inspektion durch die Inspektionsvorrichtung mit dem Greifer 36 gehalten wird. Es ist also nicht notwendig, dass der Deckel 48 in der Inspektionsvorrichtung 2 abgelegt wird. Dadurch wird der Vorgang beschleunigt. Da der Greifer den Deckel 48 nur an der Peripherie angreift, stört er den Inspektionsprozess nicht.

Wie bereits erwähnt, sind die Petrischalen 15 in der Lagervorrichtung 1 in Lagerschächten 14 gelagert. Ein vorteilhafter Lagerschacht ist in Fig. 6 und 7 dargestellt.

Der gezeigte Lagerschacht ist im Wesentlichen aus einem einzigen Blech geformt, welches in geeigneter Weise zugeschnitten und gebogen wurde. Insbesondere bildet das Blech eine vertikale Rückwand 60 und zwei vertikale Seitenwände 61. Zumindest an den Seitenwänden 61, vorzugsweise auch an der Rückwand 60, sind seitliche Ablagen 63 zur Aufnahme der Petrischalen angeordnet. Diese werden von abgebogenen Zungen des Blechs der Rück- und Seitenwände gebildet.

Die Rückwand 60 geht jeweils über einen Übergangsbereich 64 in jede Seitenwand 61 über. Dieser Übergangsbereich verläuft in der Ausführung nach Fig. 6 und 7 schräg zur Seiten, so dass Volumen, welches im rückseitigen Bereich der Lagerschächte 14 von den runden Petrischalen 15 nicht benötigt wird, freigegeben werden kann. Dies erlaubt eine dichtere Packung der Lagerschächte 14 in einem Kreis, wie in Fig. 1 illustriert, indem die Lagerschächte im Kreis näher zusammenrücken können.

Da Petrischalen einen typischen Durchmesser in der Grössenordnung von 9 cm besitzen, kann der Übergangsbereich 64 einen ebenen, vertikal verlaufenden Abschnitt 66 besitzen, dessen Ausdehnung senkrecht zur Längsachse (Vertikalachse) des Lagerschachts 14 mindestens 2 cm beträgt.

Da Petrischalen rund sind, kann der Übergangsbereich 64 grundsätzlich auch gerundet verlaufen, wie dies in Fig. 7 unter Bezugsziffer 66' gestrichelt dargestellt ist. In diesem Falle ist, wenn der Krümmungsradius des Übergangsbereichs 64 ungefähr jenem der Petrischalen entspricht, eine noch dichtere Packung der Lagerschächte 14 möglich, allerdings bei etwas höherem Herstellungsaufwand. In diesem Falle kann der Krümmungsradius des Übergangsbereichs im Mittel mindestens 2 cm betragen, vorzugsweise mindestens 5 cm.

Wie aus Fig. 6 und 7 weiter ersichtlich, gehen auch der Boden 67 und die Decke 68 jedes Lagerschachts 14 einstückig in die Rückwand 60 über und sind durch Biegen aus dem gleichen Blech wie die Rückwand 60 und die Seitenwände 61 gefertigt.

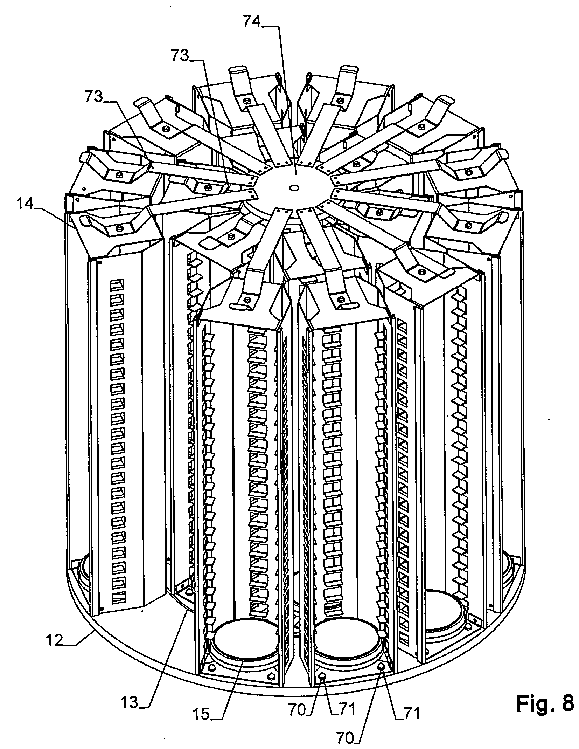

Die Lagerschächte 14 können in der Lagervorrichtung 1 grundsätzlich in jeder geeigneten Art befestigt werden. Eine bevorzugte Befestigungsvariante ist in Fig. 8 dargestellt. Hier sind im Boden 67 eine order mehrere Öffnungen 70 vorgesehen (vgl. Fig. 7), in welche Stifte 71 eingreifen. Die Stifte 71 sind mit der Unterlage verbunden, auf welcher der jeweilige Lagerschacht 14 steht. In der Ausführung nach Fig. 8 wird diese Unterlage von einem der Karussells 12 oder 13 gebildet. Die Stifte 71 sind nach oben konisch zulaufend.

Von der Oberseite werden die Lagerschächte von federnden, radial verlaufenden Zungen 73 gehalten, die an einem Mittelbereich 74 der Lagervorrichtung 1 befestigt sind. Die Zungen 73 sind vorteilhaft federnd vorgespannt und drücken von oben auf die Lagerschächte 14. Die Zungen 73 können mit den Lagerschächten 14 verschraubt sein.

Fig. 9 zeigt eine zweite Ausführung der Transfervorrichtung 3. Sie unterscheidet sich von jener gemäss Fig. 1 dadurch, dass zwei Übergabestationen 4 mit jeweils einem eigenen Transfertisch 26 übereinander vorgesehen sind. Diese sind am Lift 30 vertikal verfahrbar, vorzugsweise mit einem gemeinsamen Liftantrieb 31. Jede der Übergabestationen 4 kann auf die Höhe gefahren werden, in welcher sie mit dem Greifer 36 und der Umschlagvorrichtung 5 zusammenwirken kann. Somit kann z.B. eine erste Petrischale analysiert werden, während ihr Deckel in der einen Übergabestation 4 liegt, während die Umschlagvorrichtung 5 der anderen Übergabestation 4 einen Petrischale entnimmt oder ihr eine Petrischale zuführt.

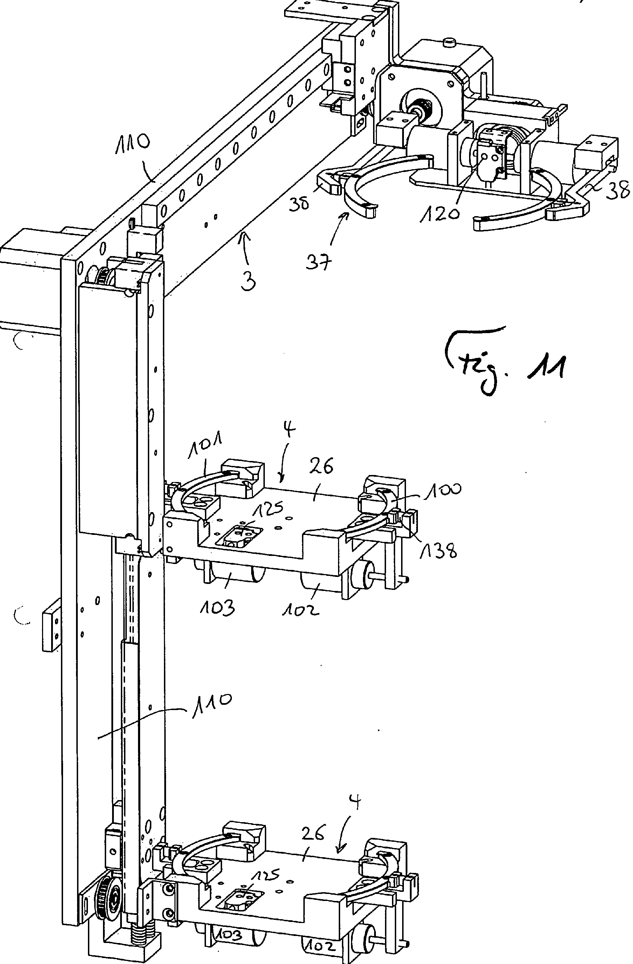

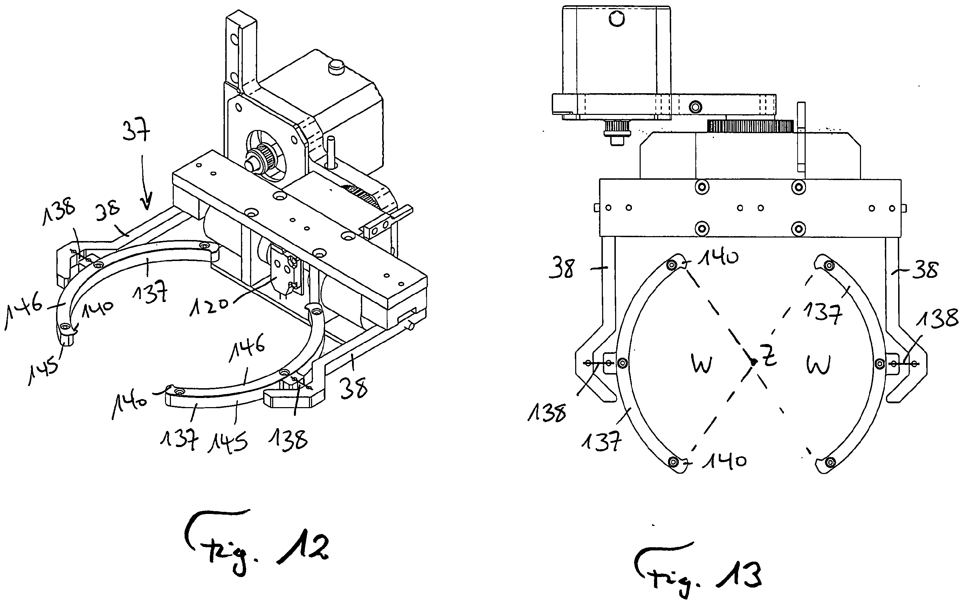

Bei einer bevorzugten Ausführung gemäss Figur 11 ist an der mindestens einen Übergabestation 4, wobei in dieser Figur zwei Übergabestationen 4 dargestellt sind, mindestens ein Fixierelement 100 für den Deckel 49 der Petrischale vorgesehen. Das Fixierelement ist so ausgeführt, dass durch dieses der Deckel gesteuert zeitweise fixierbar ist. Damit kann verhindert werden, dass ein allenfalls am Boden der Petrischale "klebender" Deckel vom Greifer mitgenommen wird, wenn der Greifer den Boden abhebt. Im gezeigten Beispiel sind an jeder Übergabestation 4 bzw. an deren Transfertisch 26 jeweils zwei Fixierelemente 100 und 101 angeordnet, welche ähnlich wie die Finger des Greifers für den Boden der Petrischale, den Deckel an seinem Rand angetrieben und gesteuert kontaktieren können und ihn damit fixieren. In der Figur sind die Antriebe 102 und 103 für die Fixierelemente 100 bzw. 101 ersichtlich, welche durch die Steuerung der Vorrichtung so betätigt werden, dass die Fixierelemente den Deckel festhalten bzw.fixieren, wenn der Greifer den Boden greift und abhebt. Die Fixierelemente sind bevorzugt bogenförmig und sie können in der Horizontalebene bzw. in einer Ebene koplanar zur Ebene des Transfertischs schwenkbar sein, um sich an den Deckel anpassen zu können. Dabei kann eine Schwenkbarkeit mit einer Blattfeder bewirkt werden, wie dies nachfolgend bei bevorzugten Fingern des Greifers bei den Figuren 12 und 13 noch erläutert wird.

In der Figur 11 ist ferner gezeigt, dass die Transfervorrichtung 3 für die Übergabestation 4, insbesondere für beide Übergabestationen 4, und den Greifer 36 eine gemeinsame Trägerplatte 110 aufweist, an welcher die Übergabestation 4 oder die Übergabestationen 4 und der Greifer 36 angeordnet sind, wobei dies insbesondere eine einstückige Trägerplatte und insbesondere L-förmige Trägerplatte ist. Dies ergibt eine sehr einfache Montage und hält die genannten Teile der Vorrichtung auf einfache Weise in genau definierter Lage zueinander, die sich nicht verstellen kann, was für die Wiederholgenauigkeit der Abläufe wichtig ist.

Bevorzugt ist es weiter, dass die Transfervorrichtung 3 im Bereich des Greifers 36 einen gesteuert zeitweise abschaltbaren optischen Sensor 120 aufweist, der zur Detektion des Vorhandenseins bzw. Nichtvorhandenseins der Petrischale im Greifer ausgestaltet und angeordnet ist. Der Sensor ist für die Abgabe von entsprechenden Signalen an die Steuerung der Vorrichtung vorgesehen. Die Abschaltung des optischen Sensors durch die Steuerung ermöglicht es, eine Störung der Inspektion der Petrischale durch den Lichteinfluss des Sensors zu vermeiden.

Bevorzugt ist weiter an jeder Übergabestation 4 ein Sensor 125 für die Präsenz bzw. Nichtpräsenz des Deckels in der Übergabestation vorgesehen, was insbesondere für die Steuerung der Fixierelemente 100 und 101 vorgesehen ist.

Die Vorrichtung ist bevorzugt so ausgebildet dass beim Greifer 36 dessen Grundstellung der greifende Zustand ist, so dass bei einer Störung, z.B. bei einem kurzzeitigen Stromunterbruch, das vom Greifer gehaltene Laborobjekt bzw. der Boden der Petrischale trotzdem festgehalten wird. Der Antrieb des Greifers wirkt somit derart, dass er den Greifer zum Loslassen antreibt und dass zum Festhalten der Antrieb nicht aktiv zu sein braucht. Bei den Fixierelementen 100 und 101 ist demgegenüber die Grundstellung das Offen sein, so dass ein Laborobjekt jedenfalls abgelegt bzw. auf dem Tisch 26 platziert werden kann und nur das Fixieren des aktiven Antriebs bedarf.

An Hand der Figuren 12 und 13 werden bevorzugte Ausführungen des Greifers erläutert, wobei der Greifer 36 mindestens zwei Finger 37 aufweist, welche je an einem Schaft 38 angeordnet sind, wobei die Schäfte gesteuert angetrieben aufeinander zu und voneinander weg bewegbar sind, wie dies schon erläutert worden ist. Ersichtlich ist, dass jeder Finger 37 in einer Horizontalebene schwenkbar am zugehörigen Schaft befestigt ist, was durch die gebogenen Pfeile in Figur 12 symbolisiert ist. Dabei ist es insbesondere vorgesehen, dass jeder Finger sich selbständig in eine unverschwenkte zentrierte Grundstellung bewegen kann, was auf eine einfache Weise eine gute Anpassung an jede neue zu greifende Schale ermöglicht. Dies ist bevorzugt so gelöst, dass der jeweilige Finger 37 mittels einer vertikal stehenden Blattfeder 138 an einer Stelle am zugehörigen Schaft befestigt ist. Die Blattfeder erlaubt die Schwenkbewegung in der Horizontalebene und nur in der Horizontalebene und bewirkt die Rückstellung.

Auch bei dieser Ausführung sind die Finger 37 des Greifers 36 für eine im Wesentlichen punktförmige Berührung der Petrischale ausgestaltet und angeordnet, wobei insbesondere eine punktförmige Berührung mittels der Endbereiche 140 der Finger bevorzugt ist, wie dies in den Figuren ersichtlich ist. Dabei ist bevorzugt, dass der Greifer 36 derart ausgestaltet ist, dass die Grösse des Winkels W vom Zentrum Z des Greifbereichs zu den äussersten, zur Berührung der Petrischale vorgesehenen und angeordneten Berührungsteilen der Finger 37 des Greifers 36 80 Winkelgrad bis 120 Winkelgrad beträgt und vorzugsweise 85 Grad bis 100 Grad beträgt und vorzugsweise ca. 90 Grad beträgt. Insbesondere der Wert von ca. 90 Grad ist für ein sicheres Greifen und Festhalten und Loslassen bevorzugt.
Weiter ist es vorteilhaft für die Ausführung und die Wartung, wenn die Finger 37 des Greifers 36 von einem Basisteil 145 und einem an diesem lösbar befestigten Wechselteil 146 gebildet sind. Das Wechselteil ist dabei für die punktförmige Berührung ausgestaltet. Das Basisteil kann aus einem Leichtmetall, insbesondere Aluminium, gebildet sein und das Wechselteil aus Stahl, insbesondere Federstahl.

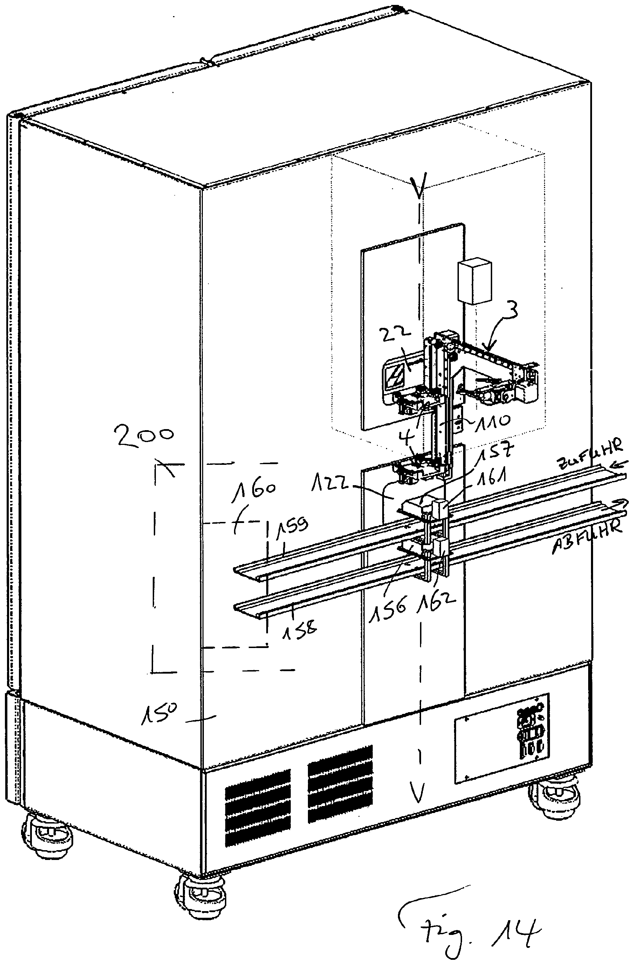

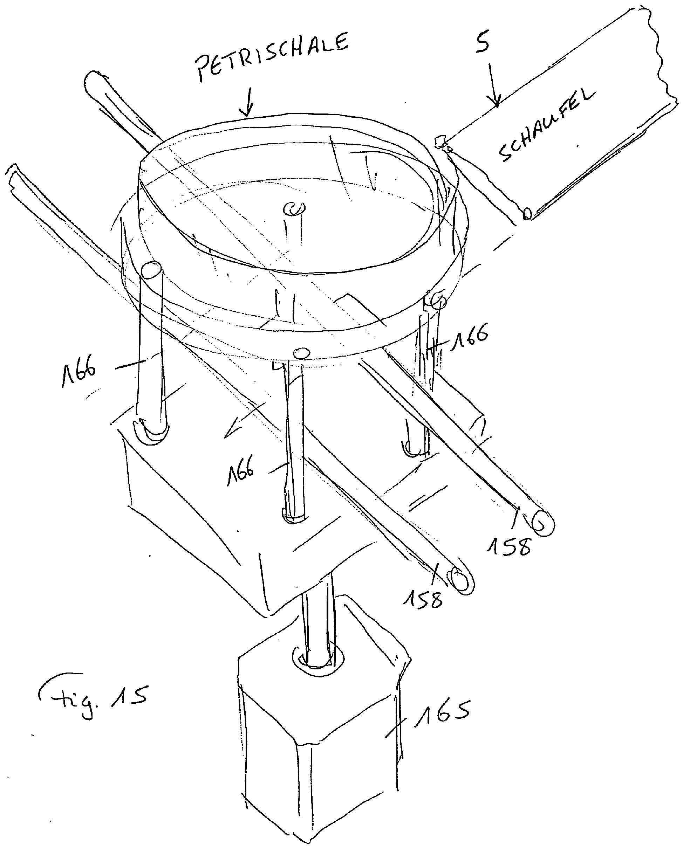

Bevorzugt ist die Vorrichtung in eine weitere Laborumgebung eingebunden und in diesem Zusammenhang ist es bevorzugt, wenn die Vorrichtung, wie in Figur 14 dargestellt, so ausgebildet ist, dass die Lagervorrichtung 1 an ihrer Wand 150, an welcher die Transfervorrichtung 3 befindlich ist, eine zweite Hilfstüre 122 aufweist, durch welche mittels der Umschlagsvorrichtung 5 und einer ausserhalb der Lagervorrichtung 1 an deren genannter Wand 150 befindlichen Transportanordnung 155 die Zufuhr bzw. Abfuhr von Petrischalen in bzw. aus der Lagervorrichtung bewirkbar ist, wobei die zweite Hilfstüre 122 insbesondere in derselben Vertikalachse V liegt wie die erste Hilfstüre 22, insbesondere unterhalb der ersten Hilfstüre. Dies erlaubt die Beschickung mit und die Entsorgung von Petrischalen im Zusammenspiel mit der Laborumgebung und der Inspektion und erbringt einen Geschwindigkeitsvorteil bei der Bewirtschaftung der Laborobjekte bzw. Petrischalen. Wie dargestellt, weist die Transportanordnung 155 zwei vertikal übereinander liegende steuerbar angetriebene Hebeeinrichtungen 156, 157 auf, durch welche Petrischalen von zwei übereinander angeordneten Förderbändern 158, 159 unabhängig voneinander abhebbar bzw. auf diesen absetzbar sind. Eine Hebeeinrichtung 156 ist beispielhaft in Figur 15 dargestellt. Gemäss Figur 14 dient diese Hebeeinrichtung zur Abfuhr von Petrischalen, welche von der Schaufel der Umschlagvorrichtung 5 durch die zweite Türe 122 aus der Lagervorrichtung ausgegeben werden. Durch deren unterseitigen Antrieb 165 können vier Säulen 166 angehoben werden und können die auf der Schaufel liegende Petrischale von unten her stützen, so dass die Schaufel der Umschlagvorrichtung 5 wieder zurückgezogen werden kann und die Petrischale auf den Säulen 166 aufliegt. Diese werden dann wieder abgesenkt werden und platzieren die Schale auf dem Förderband 158, das hier aus zwei separaten, synchron angetriebenen Riemen gebildet ist. Eine Steuerung und ein Antrieb 160 des Förderbands 158, welche Elemente zur übergeordneten Laborumgebung 200 gehören, die in Figur 14 angedeutet ist, führen dann die Petrischale z.B. zur Entsorgung ab. Für die andere Hebeeinrichtung 157 gilt für die Zufuhr von Petrischalen in die Lagervorrichtung 1 grundsätzlich dasselbe Vorgehen, wobei aber hier eine Petrischale von dem Förderband 159 zugeführt, von der Hebeeinrichtung 157 angehoben von der Schaufel der Umschlagvorrichtung 5 unterfahren und durch Absenken der Hebeeinrichtung von der Schaufel der Umschlagvorrichtung 5 übernommen und dann in die Lagervorrichtung 1 durch die zweite Türe eingefahren wird. Für die Steuerung dieser Vorgänge ist es bevorzugt, dass die Hebeeinrichtung jeweils einen Sensor aufweist, durch den das Vorhandensein bzw. Nichtvorhandensein einer Petrischale feststellbar ist.

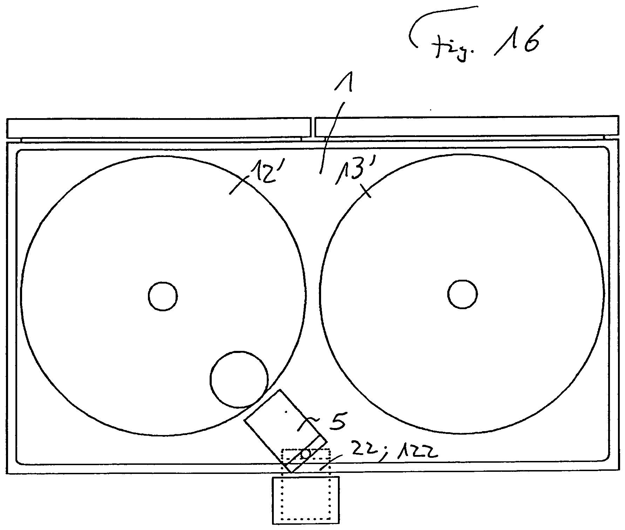

Gemäss Figur 16 ist es bevorzugt, wenn in der Lagervorrichtung zwei getrennt voneinander und nebeneinander angeordnete Karussells 12', 13' vorgesehen sind, welche von einer gemeinsamen Umschlagvorrichtung 5 bedienbar sind.

Die erläuterte Laborvorrichtung mit einer Vorrichtung erfindungsgemässen Vorrichtung wobei die Laborvorrichtung eine Steuerung und zwei übereinander liegende, steuerbar angetriebene Förderbänder 158, 159 aufweist, wobei die Förderbänder derart angeordnet und steuerbar sind, dass sie zum Zusammenwirken mit den Hebeeinrichtungen 156, 157 bestimmt sind, um Petrischalen in die bzw. aus der Lagervorrichtung (1) der Vorrichtung zuzuführen bzw. abzuführen ermöglicht eine besonders effiziente und rasche Handhabung der Petrischalen.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Vorrichtung zur Lagerung und Handhabung von Petrischalen (15), wobei die Petrischalen (15) einen Boden (48) und einen Deckel (49) aufweisen, wobei an einem Umfang des Bodens (48) eine Bodenwand (53) angeordnet ist, umfassend
eine Lagervorrichtung (1) zum Lagern der Petrischalen (15) mit dem Deckel (49) nach unten,
einen Greifer (36), mit welchem jeweils eine Petrischale (15) ohne den Deckel (49) lateral an der Bodenwand (53) ergreifbar ist,
**gekennzeichnet durch**
eine Inspektionsvorrichtung (2) zum automatisierten Inspizieren von Petrischalen (15) ohne Deckel (49) und
eine Transfervorrichtung (3) zum Transferieren der Petrischalen (15) zwischen der Lagervorrichtung (1) und der Inspektionsvorrichtung (2),
wobei die Transfervorrichtung (3) mindestens eine Übergabestation (4) zur Aufnahme einer Petrischale (15) mit dem Deckel (49) nach unten aufweist, von welcher jeweils eine Petrischale (15) ohne den Deckel (49) vom Greifer (36) ergreifbar ist, und
wobei die Transfervorrichtung (3) einen Wendeantrieb (34a) aufweist, mit welchem der Greifer (36) mit einem aufgenommenen Boden (48) einer Petrischale (15) vertikal oberhalb des in der Übergabestation (4) liegenden Deckels (49) wendbar ist.

2. Vorrichtung nach Anspruch 1, wobei mit dem Wendeantrieb (34a) der Greifer (36) mit dem aufgenommenen Boden (48) vertikal oberhalb des in der Übergabestation (4) liegenden Deckels (49) um mehr als 90° wendbar ist.

3. Vorrichtung nach einem der Ansprüche 1 oder 2, wobei die Vorrichtung einen Liftantrieb (31) aufweist, mit welchem der Greifer (36) relativ zur Übergabestation (4) vertikal verschiebbar ist.

4. Vorrichtung nach einem der Ansprüche 1 bis 3, wobei die Transfervorrichtung (3) einen Horizontalantrieb (35) aufweist, mit welchem der Greifer (36) von der Transfervorrichtung (3) zur Inspektionsvorrichtung (2) und zurück bewegbar ist.

5. Vorrichtung nach einem der Ansprüche 1 bis 4, wobei die Vorrichtung zwei Übergabestationen (4) aufweist, welche beide verfahrbar sind, und insbesondere wobei ein gemeinsamer Liftantrieb (31) für beide Übergabestationen (4) vorgesehen ist.

6. Vorrichtung nach einem der vorangehenden Ansprüche, wobei die Vorrichtung dazu ausgestaltet ist, den Deckel (49) während der Inspektion durch die Inspektionsvorrichtung (2) mit dem Greifer (36) zu halten.

7. Vorrichtung nach einem der vorangehenden Ansprüche, wobei die Lagervorrichtung (1) eine Vielzahl von Lagerplätzen für Petrischalen (15) mit dem Deckel (49) nach unten aufweist sowie eine Umschlagvorrichtung (5), um die Petrischalen (15) zwischen den Lagerplätzen und der Transfervorrichtung (3) zu transportieren, und insbesondere wobei die Umschlagvorrichtung zum Transferieren der Petrischalen (15) zwischen den Lagerplätzen und der Übergabestation (4) ohne Wenden der Petrischalen (15) ausgestaltet ist.

8. Vorrichtung nach Anspruch 7, wobei die Lagerplätze von einer Vielzahl von Lagerschächten (14) gebildet werden, wobei jeder Lagerschacht (14) mehrere Lagerplätze übereinander aufweist, und insbesondere wobei die Lagerschächte auf mindestens einem Karussell (12, 13) angeordnet sind.

9. Vorrichtung nach einem der Ansprüche 7 oder 8, wobei die Umschlagvorrichtung (5) zwei vertikal verfahrbare Wagen (16, 16b) aufweist, und insbesondere wobei die Wagen (16, 16b) an einer gemeinsamen Liftsäule (16a) übereinander angeordnet sind.

10. Vorrichtung nach einem der vorangehenden Ansprüche, wobei der Greifer (36) zwei gegeneinander bewegliche Finger (37) aufweist, welche mehrere, vorzugsweise insgesamt drei oder, gegeneinander gerichtete Erhöhungen (43) zur Beaufschlagung der Wand der Petrischale (15) aufweisen.

11. Vorrichtung nach Anspruch 10, wobei mindestens einer der Finger (37) eine Federung (39) aufweist, welche einen Basisteil (42) trägt, wobei am Basisteil (42) mindestens eine der Erhöhungen (43) angeordnet ist.

12. Vorrichtung nach einem der vorangehenden Ansprüche, wobei die Lagervorrichtung (1) mindestens einen Lagerschacht (14) aufweist, wobei jeder Lagerschacht (14) mehrere Lagerplätze zur Aufnahme jeweils eines Laborobjekts übereinander aufweist, wobei in einem Boden (67) jedes Lagerschachts (14) mindestens eine Öffnung (70) angeordnet ist, in welche mindestens ein Stift (71) einer Unterlage (12, 13) des Lagerschachts eingreift, und wobei die Lagerschächte (14) von oben mittels federnder Zungen (73) gehalten sind.

13. Vorrichtung nach Anspruch 12, wobei die Lagervorrichtung mindestens ein Karussell (12, 13) aufweist, welches die Unterlage für die Lagerschächte bildet, wobei die Lagerschächte in mindestens einem Kreis auf dem mindestens einen Karussell (12, 13) angeordnet sind.

14. Vorrichtung nach Anspruch 13, wobei die Zungen (73) radial verlaufen und an einem Mittelbereich (74) der Lagervorrichtung befestigt sind.

15. Vorrichtung nach einem der Ansprüche 13 oder 14, wobei die Zungen (73) federnd vorgespannt sind und von oben auf die Lagerschächte (14) drücken.

16. Vorrichtung nach einem der vorangehenden Ansprüche, wobei die Lagervorrichtung (1) einen Lagerschacht (14) aufweist, wobei der Lagerschacht (14) eine Rückwand (60) und zwei Seitenwände (61) aufweist, und wobei zumindest an den Seitenwänden (61) seitliche Ablagen (63) zur Aufnahme der Petrischalen (15) angeordnet sind, **dadurch gekennzeichnet, dass** die Rückwand (60) über jeweils einen Übergangsbereich (64) in jede Seitenwand (61) übergeht, wobei der Übergangsbereich (64) gerundet ist und/oder schräg zu der Rückwand (60) und der an ihn angrenzenden Seitenwand (61) verläuft.

17. Vorrichtung nach Anspruch 16, wobei der Zwischenbereich mit einem Krümmungsradius von mindestens 2 cm, vorzugsweise mindestens 5 cm, gerundet ist.

18. Vorrichtung nach Anspruch 16, wobei der Zwischenbereich einen im Wesentlichen ebenen Abschnitt aufweist, welcher senkrecht zu einer Längsachse des Lagerschachts eine Ausdehnung von mindestens 2 cm aufweist.

19. Vorrichtung nach einem der Ansprüche 16 bis 18, wobei auch an der Rückwand Ablagen (63) zur Aufnahme der Petrischalen (15) angeordnet sind.

20. Vorrichtung nach einem der Ansprüche 1 bis 11, wobei mindestens ein Fixierelement (100) für den Deckel (49) der Petrischale vorgesehen ist, durch welches der Deckel (49) gesteuert zeitweise fixierbar ist, wobei das mindestens eine Fixierelement, das insbesondere ein bogenförmiges Fixierelement ist, insbesondere am Transfertisch (26) der Übergabestation (4) angeordnet ist, und wobei insbesondere zwei, vorzugsweise bogenförmige, Fixierelemente (100, 101),vorgesehen sind, die vorzugsweise am Transfertisch (26) der Übergabestation angeordnet sind.

21. Vorrichtung nach einem der Ansprüche 1 bis 11 oder 20, wobei die Transfervorrichtung (3) für die Übergabestation (4), insbesondere für beide Übergabestationen (4), und den Greifer (36) eine gemeinsame Trägerplatte (110) aufweist, an welcher die Übergabestation(4) oder die Übergabestationen (4) und der Greifer (36) angeordnet sind, wobei dies insbesondere eine einstückige Trägerplatte und insbesondere L-förmige Trägerplatte ist.

22. Vorrichtung nach einem der Ansprüche 1 bis 11 oder 20 bis 21, wobei die Transfervorrichtung (3) im Bereich des Greifers (36) einen gesteuert zeitweise abschaltbaren optischen Sensor (120) aufweist, der zur Detektion des Vorhandenseins bzw. Nichtvorhandenseins der Petrischale im Greifer ausgestaltet und angeordnet ist.

23. Vorrichtung nach einem der Ansprüche 1 bis 11 oder 20 bis 22, wobei an jeder Übergabestation (4) ein Sensor (125) für die Präsenz bzw. Nichtpräsenz des Deckels in der Übergabestation vorgesehen ist.

24. Vorrichtung nach einem der Ansprüche 1 bis 11 oder 20 bis 23, wobei der Greifer derart ausgestaltet ist, dass seine Grundstellung der greifende Zustand ist, und insbesondere wobei das mindestens eine Fixierelement (100, 101) derart ausgestaltet ist, dass seine Grundstellung die offene, nichtfixierende Stellung ist.

25. Vorrichtung nach einem der Ansprüche 1 bis 11 oder 20 bis 24, wobei der Greifer (36) mindestens zwei Finger (37) aufweist, welche je an einem Schaft (38) angeordnet sind, wobei die Schäfte gesteuert angetrieben aufeinander zu und voneinander weg bewegbar sind, und wobei jeder Finger (37) in einer Horizontalebene schwenkbar am zugehörigen Schaft befestigt ist, insbesondere wobei jeder Finger sich selbständig in eine unverschwenkte Grundstellung zentrierend am Schaft befestigt ist, und wobei insbesondere jeder Finger (37) mit seiner Rückseite (137) über eine Blattfeder (138) am Schaft befestigt ist.

26. Vorrichtung nach einem der Ansprüche 1 bis 11 oder 20 bis 25, wobei die Finger (37) des Greifers (36) für eine im Wesentlichen punktförmige Berührung der Petrischale ausgestaltet und angeordnet sind, insbesondere für eine punktförmige Berührung mittels der Endbereiche (140) der Finger.

27. Vorrichtung nach einem der Ansprüche 1 bis 11 oder 20 bis 26, wobei die Finger (37) des Greifers (36) von einem Basisteil (145) und einem an diesem lösbar befestigten Wechselteil (146), welches Wechselteil für die punktförmige Berührung ausgestaltet ist, gebildet sind, und wobei insbesondere das Basisteil aus einem Leichtmetall, insbesondere Aluminium, gebildet ist und das Wechselteil aus Stahl, insbesondere Federstahl gebildet ist.

28. Vorrichtung nach einem der Ansprüche 1 bis 11 oder 20 bis 28, wobei der Greifer (36) derart ausgestaltet ist, dass die Grösse des Winkels W vom Zentrum Z des Greifbereichs zu den äussersten, zur Berührung der Petrischale vorgesehenen und angeordneten Berührungsteile der Finger (37)des Greifers (36) 80 Grad bis 120 Grad beträgt und vorzugsweise 85 Grad bis 100 Grad beträgt und vorzugsweise ca. 90 Grad beträgt.

29. Vorrichtung nach einem der Ansprüche 1 bis 11 oder 20 bis 28, wobei die Lagervorrichtung (1) an ihrer Wand (150), an welcher die Transfervorrichtung (3) befindlich ist, eine zweite Hilfstüre (122) aufweist, durch welche mittels der Umschlagsvorrichtung (5) und einer ausserhalb der Lagervorrichtung (1) an deren genannter Wand (150) befindlichen Transportanordnung (155) die Zufuhr bzw. Abfuhr von Petrischalen in bzw. aus der Lagervorrichtung bewirkbar ist, wobei die zweite Hilfstüre (122) insbesondere in derselben Vertikalachse V liegt wie die erste Hilfstüre (22), insbesondere unterhalb der ersten Hilfstüre.

30. Vorrichtung nach einem der Ansprüche 1 bis 11 oder 20 bis 29, wobei die Transportanordnung (155) zwei vertikal übereinander liegende steuerbar angetriebene Hebeeinrichtungen (156, 157) umfasst, durch welche Petrischalen von zwei übereinander angeordneten Förderbändern (158, 159) unabhängig voneinander abhebbar bzw. auf diesen absetzbar sind.

31. Vorrichtung nach einem der Ansprüche 1 bis 11 oder 20 bis 30, wobei die Hebeeinrichtung jeweils einen Sensor aufweist, durch den das Vorhandensein bzw. Nichtvorhandensein einer Petrischale feststellbar ist.

32. Vorrichtung nach einem der Ansprüche 1 bis 11 oder 20 bis 31, wobei in der Lagervorrichtung zwei getrennt voneinander und nebeneinander angeordnete Karussells (12', 13') vorgesehen sind, welche von einer gemeinsamen Umschlagvorrichtung (5) bedienbar sind.

33. Laborvorrichtung mit einer Vorrichtung nach einem der Ansprüche 1 bis 11 oder 20 bis 32, wobei die Laborvorrichtung eine Steuerung und zwei übereinander liegende, steuerbar angetriebene Förderbänder (158, 159) aufweist, wobei die Förderbänder derart angeordnet und steuerbar sind, dass sie zum Zusammenwirken mit den Hebeeinrichtungen (156, 157) bestimmt sind, um Petrischalen in die bzw. aus der Lagervorrichtung (1) der Vorrichtung zuzuführen bzw. abzuführen.

## Claims

1. Device for storing and handling Petri dishes (15), wherein the Petri dishes (15) have a base (48) and a lid (49), wherein a base wall (53) is arranged on a circumference of the base (48), comprising
a storage device (1) for storing the Petri dishes (15) with the lid (49) downwards,
a gripper (36) by means of which one Petri dish (15) at a time without the lid (49) can be grasped laterally on the base wall (53),
**characterized by**
an inspection device (2) for the automatic inspection of Petri dishes (15) without a lid (49) and
a transfer device (3) for transferring the Petri dishes (15) between the storage device (1) and the inspection device (2),
wherein the transfer device (3) has at least one transfer station (4) for holding a Petri dish (15) with the lid (49) downwards, from which one Petri dish (15) at a time can be grasped without the lid (49) by the gripper (36) and
wherein the transfer device (3) has a turning drive (34a), by means of which the gripper (36) with a held base (48) of a Petri dish (15) can be turned over vertically above the lid lying in the transfer station.

2. Device according to claim 1, wherein with the turning drive (34a) the gripper (36) with a held base (48) of a Petri dish (15) can be turned over vertically above the lid (49) lying in the transfer station (4) by more than 90°.

3. Device according to one of claims 1 or 2, wherein the device has a lift drive (31), with which the gripper (36) can be displaced vertically relative to the transfer station (4).

4. Device according to one of claims 1 to 3, wherein the transfer device (3) has a horizontal drive (35), with which the gripper (36) can be moved by the transfer device (3) to the inspection device (2) and back.

5. Device according to one of claims 1 to 4, wherein the device has two transfer stations (4), both of which are moveable, and in particular wherein a common lift drive (31) for both transfer stations (4) is provided.

6. Device according to one of the preceding claims, wherein the device is designed to hold the lid (49) with the gripper (36) during the inspection by the inspection device (2).

7. Device according to one of the preceding claims, wherein the storage device (1) has a plurality of storage locations for Petri dishes (15) with the lid (49) downwards and a handling device (5) in order to transport the Petri dishes (15) between the storage locations and the transfer device (3), and in particular wherein the handling device is designed for transferring the Petri dishes (15) between the storage locations and the transfer station (4) without turning over the Petri dishes (15).

8. Device according to claim 7, wherein the storage locations are formed by a plurality of storage racks (14), wherein each storage rack (14) has several storage locations one above the other, and in particular wherein the storage racks are arranged on at least one carousel (12, 13).

9. Device according to one of claims 7 or 8, wherein the handling device (5) has two vertically moveable carriages (16, 16b), and in particular wherein the carriages (16, 16b) are arranged above one another on a common lift column (16a).

10. Device according to one of the preceding claims, wherein the gripper (36) has two fingers (37) moveable with respect to one another, which have several, preferably a total of three or, elevations (43) directed against one another for acting on the wall of the Petri dish (15).

11. Device according to claim 10, wherein at least one of the fingers (37) has a spring (39) mounting, which bears a base part (42), wherein at least one of the elevations (43) is arranged on the base part (42).

12. Device according to one of the preceding claims, wherein the storage device (1) has at least one storage rack (14), wherein each storage rack (14) has several storage locations for holding respectively one laboratory object one above the other, wherein in a base (67) of each storage rack (14) at least one opening (70) is arranged, which is engaged by at least one pin (71) of a substrate (12, 13) of the storage rack, and wherein the storage racks (14) are held from above by means of resilient tongues (73).

13. Device according to claim 12, wherein the storage device has at least one carousel (12, 13), which forms the substrate for the storage racks, wherein the storage racks are arranged in at least one circle on the at least one carousel (12, 13).

14. Device according to claim 13, wherein the tongues (73) run radially and are attached to a center region (74) of the storage device.

15. Device according to one of claims 13 or 14, wherein the tongues (73) are flexibly biased and press from above on the storage racks (14).

16. Device according to one of the preceding claims, wherein the storage device (1) has at least a storage rack (14), wherein the storage rack (14) has a rear wall (60) and two sidewalls (61), and wherein lateral storage compartments (63) for holding the Petri dishes (15) are arranged at least on the sidewalls (61), **characterized in that** the rear wall (60) merges via respectively one transition region (64) into each sidewall (61), wherein the transition region (64) is rounded and/or runs obliquely to the rear wall (60) and the sidewall (61) adjacent thereto.

17. Device according to claim 16, wherein the intermediate region is rounded with a radius of curvature of at least 2 cm, preferably at least 5 cm.

18. Device according to claim 16, wherein the intermediate region has an essentially flat section, which has an extension of at least 2 cm perpendicular to a longitudinal axis of the storage rack.

19. Device according to one of claims 16 to 18, wherein storage compartments (63) for holding the Petri dishes (15) are also arranged on the rear wall.

20. Device according to one of claims 1 to 11, wherein at least one fixing element (100) for the lid (49) of the Petri dish is provided, by which the lid (49) can be temporarily fixed in a controlled manner, wherein the at least one fixing element, which in particular is an arched fixing element, is arranged in particular on the transfer table (26) of the transfer station (4), and wherein in particular two preferably arched fixing elements (100, 101) are provided, which are preferably arranged on the transfer table (26) of the transfer station.

21. Device according to one of claims 1 to 12 or 20, wherein the transfer device (3) has a common carrier plate (110) for the transfer station (4), in particular for both transfer stations (4), and the gripper (36), on which carrier plate the transfer station (4) or the transfer stations (4) and the gripper (36) are arranged, wherein this is in particular a one-piece carrier plate and in particular is an L-shaped carrier plate.

22. Device according to one of claims 1 through 11 or 20 to 21, wherein the transfer device (3) in the region of the gripper (36) has an optical sensor (120) that can be switched off temporarily in a controlled manner, which sensor is designed and arranged for the detection of the presence or absence of the Petri dish in the gripper.

23. Device according to one of claims 1 to 11 or 20 to 22, wherein a sensor (125) for the presence or absence of the lid (49) in the transfer station is provided at each transfer station (3).

24. Device according to one of claims 1 to 11 or 20 to 23, wherein the gripper (36) is embodied such that its base position is the grasping condition and in particular wherein the at least one fixing element (100, 101) is designed such that its base position is the open, non-fixing position.

25. Device according to one of claims 1 to 11 or 20 to 24, wherein the gripper (36) has at least two fingers (37), which are arranged respectively on one body (38), wherein the bodies can be moved towards one another and away from one another in a controlled driven manner, and wherein each finger (37) is attached to the associated body in a manner pivotable in a horizontal plane, in particular wherein each finger is attached to the shaft centering itself automatically into an unpivoted base position, and wherein in particular each finger (37) is attached to the shaft by its rear side (137) via a leaf spring (138).

26. Device according to one of claims 1 to 11 or 20 to 25, wherein the fingers (37) of the gripper (36) are designed and arranged for an essentially punctiform contact of the Petri dish, in particular for a punctiform contact by means of the end regions (140) of the fingers.

27. Device according to one of claims 1 to 11 or 20 to 26, wherein the fingers (37) of the gripper (36) are formed by a base part (145) and an interchange part (146) releasably attached thereto, which interchange part is designed for the punctiform contact, and wherein in particular the base part is formed of a light metal, in particular aluminum, and the interchange part is formed of steel, in particular spring steel.

28. Device according to one of claims 1 to 11 or 20 to 28, wherein the gripper (36) is designed such that the size of the angle W from the center Z of the grasping region to the outermost contact parts, designed and arranged for contacting the Petri dish, of the fingers (37) of the gripper (36) is 80 to 120 degrees and preferably 85 to 100 degrees and preferably is approx. 90 degrees.

29. Device according to one of claims 1 to 11 or 20 through 28, wherein the storage device (1) has a second auxiliary door (122) on its wall (150), on which the transfer device (3) is found, through which auxiliary door the supply or removal of Petri dishes into or out of the storage device can be carried out by means of the handling device (5) and a transport arrangement (155) located outside the storage device (1) on the cited wall thereof, wherein the second auxiliary door (122) in particular lies in the same vertical axis V as the first auxiliary door (22), in particular below the first auxiliary door.

30. Device according to one of claims 1 to 11 or 20 to 29, wherein the transport arrangement (155) comprises two lifting devices (156, 157) lying vertically one above the other and driven in a controlled manner, by which Petri dishes, independently of one another, can be lifted from or deposited on two conveyor belts (158, 159) arranged one above the other.

31. Device according to one of claims 1 to 11 or 20 to 30, wherein the lifting device has respectively one sensor, by means of which the presence or absence of a Petri dish can be established.

32. Device according to one of claims 1 to 11 or 20 to 31, wherein two carousels (12', 13') arranged separately from one another and next to one another are provided in the storage device, which can be served by a common handling device (5).

33. Laboratory device with a device according to one of claims 1 to 11 or 20 to 32, wherein the laboratory device has a control and two conveyor belts (158, 159) lying one above the other and driven in a controlled manner, wherein the conveyor belts are arranged and can be controlled such that they are designed to interact with the lifting devices (156, 157) in order to feed Petri dishes into the storage device (1) of the device or to remove them therefrom.

## Revendications

1. Dispositif pour le stockage et la manipulation de boîtes de Pétri (15), dans lequel les boîtes de Pétri (15) présentent un fond (48) et un couvercle (49), dans lequel une paroi de fond (53) est disposée sur une circonférence du fond (48), comprenant
un dispositif de stockage (1) pour le stockage des boîtes de Pétri (15) avec le couvercle (49) vers le bas,
une pince (36) au moyen de laquelle une boîte de Pétri (15) à la fois sans le couvercle (49) peut être saisie latéralement sur la paroi du fond (53),
**caractérisé par**
un dispositif d'inspection (2) pour l'inspection automatique des boîtes de Pétri (15) sans couvercle (49) et
un dispositif de transfert (3) pour transférer les boîtes de Pétri (15) entre le dispositif de stockage (1) et le dispositif d'inspection (2),
dans lequel le dispositif de transfert (3) comporte au moins une station de transfert (4) pour tenir une boîte de Petri (15) avec le couvercle (49) vers le bas, à partir de laquelle une boîte de Petri (15) à la fois peut être saisie sans le couvercle (49) par la pince (36) et
dans lequel le dispositif de transfert (3) comporte un entraînement rotatif (34a), au moyen duquel la pince (36) avec un fond maintenu (48) d'une boîte de Pétri (15) peut être retournée verticalement au-dessus du couvercle se trouvant dans la station de transfert.

2. Dispositif selon la revendication 1, dans lequel, grâce à l'entraînement en rotation (34a), la pince (36) avec un fond maintenu (48) d'une boîte de Pétri (15) peut être retournée de plus de 90° verticalement au-dessus du couvercle (49) se trouvant dans la station de transfert (4).

3. Dispositif selon l'une des revendications 1 ou 2, le dispositif comportant un entraînement de levage (31), avec lequel la pince (36) peut être déplacée verticalement par rapport à la station de transfert (4).

4. Dispositif selon l'une des revendications 1 à 3, dans lequel le dispositif de transfert (3) présente un entraînement horizontal (35), avec lequel la pince (36) peut être déplacée par le dispositif de transfert (3) vers le dispositif d'inspection (2) et inversement.

5. Dispositif selon l'une des revendications 1 à 4, dans lequel le dispositif présente deux stations de transfert (4), qui sont toutes deux mobiles, et en particulier dans lequel il est prévu un entraînement de levage (31) commun pour les deux stations de transfert (4) .

6. Dispositif selon l'une des revendications précédentes, dans lequel le dispositif est conçu pour maintenir le couvercle (49) avec la pince (36) pendant l'inspection par le dispositif d'inspection (2).

7. Dispositif selon l'une des revendications précédentes, dans lequel le dispositif de stockage (1) comporte plusieurs emplacements de stockage pour des boîtes de Pétri (15) avec le couvercle (49) vers le bas et un dispositif de manipulation (5) afin de transporter les boîtes de Pétri (15) entre les emplacements de stockage et le dispositif de transfert (3), et en particulier dans lequel le dispositif de manipulation est conçu pour transférer les boîtes de Pétri (15) entre les emplacements de stockage et la station de transfert (4) sans retourner les boîtes de Pétri (15).

8. Dispositif selon la revendication 7, dans lequel les emplacements de stockage sont formés par une pluralité de rayonnages de stockage (14), dans lequel chaque rayonnage de stockage (14) présente plusieurs emplacements de stockage les uns au-dessus des autres, et en particulier dans lequel les rayonnages de stockage sont disposés sur au moins un carrousel (12, 13).

9. Dispositif selon l'une des revendications 7 ou 8, le dispositif de manutention (5) présentant deux chariots (16, 16b) mobiles verticalement, et en particulier les chariots (16, 16b) étant disposés les uns au-dessus des autres sur une colonne d'élévation commune (16a).

10. Dispositif selon l'une des revendications précédentes, dans lequel la pince (36) comporte deux doigts (37) mobiles l'un par rapport à l'autre, qui présentent plusieurs, de préférence trois ou, des élévations (43) dirigées les unes contre les autres pour agir sur la paroi de la boîte de Pétri (15).

11. Dispositif selon la revendication 10, dans lequel au moins l'un des doigts (37) présente un montage à ressort (39), qui porte une partie de base (42), dans lequel au moins l'une des élévations (43) est disposée sur la partie de base (42).

12. Dispositif selon l'une des revendications précédentes, dans lequel le dispositif de stockage (1) présente au moins un rayonnage de stockage (14), dans lequel chaque rayonnage de stockage (14) présente plusieurs emplacements de stockage pour maintenir respectivement un objet de laboratoire l'un au-dessus de l'autre, dans lequel dans un fond (67) de chaque rayonnage de stockage (14) est disposée au moins une ouverture (70), dans laquelle s'engage au moins une broche (71) d'un substrat (12, 13) du rayonnage de stockage, et dans lequel les rayonnages de stockage (14) sont maintenus par le haut au moyen de languettes élastiques (73).

13. Dispositif selon la revendication 12, le dispositif de stockage présentant au moins un carrousel (12, 13), qui forme le substrat pour les rayonnages de stockage, les rayonnages de stockage étant disposées en au moins un cercle sur le au moins un carrousel (12, 13).

14. Dispositif selon la revendication 13, dans lequel les languettes (73) s'étendent radialement et sont fixées à une zone centrale (74) du dispositif de stockage.

15. Dispositif selon l'une des revendications 13 ou 14, dans lequel les languettes (73) sont sollicitées de manière flexible et appuient par le haut sur les rayonnages de stockage (14).

16. Dispositif selon l'une des revendications précédentes, dans lequel le dispositif de stockage (1) présente au moins un rayonnage de stockage (14), dans lequel le rayonnage de stockage (14) présente une paroi arrière (60) et deux parois latérales (61), et dans lequel des compartiments de stockage latéraux (63) destinés à recevoir les boîtes de Petri (15) sont disposés au moins sur les parois latérales (61), **caractérisé en ce que** la paroi arrière (60) se prolonge par une zone de transition (64) dans chaque paroi latérale (61), la zone de transition (64) étant arrondie et/ou s'étendant obliquement par rapport à la paroi arrière (60) et à la paroi latérale (61) qui lui est adjacente.

17. Dispositif selon la revendication 16, dans lequel la zone intermédiaire est arrondie avec un rayon de courbure d'au moins 2 cm, de préférence d'au moins 5 cm.

18. Dispositif selon la revendication 16, dans lequel la zone intermédiaire a une section essentiellement plate, qui a une extension d'au moins 2 cm perpendiculairement à un axe longitudinal du rayonnage de stockage.

19. Dispositif selon l'une des revendications 16 à 18, dans lequel des compartiments de stockage (63) destinés à contenir les boîtes de Pétri (15) sont également disposés sur la paroi arrière.

20. Dispositif selon l'une des revendications 1 à 11, dans lequel il est prévu au moins un élément de fixation (100) pour le couvercle (49) de la boîte de Pétri, par lequel le couvercle (49) peut être fixé temporairement de manière contrôlée, dans lequel le au moins un élément de fixation, qui est en particulier un élément de fixation arqué, est disposé en particulier sur la table de transfert (26) de la station de transfert (4), et dans lequel il est prévu en particulier deux éléments de fixation (100, 101) de préférence arqués, qui sont disposés de préférence sur la table de transfert (26) de la station de transfert.

21. Dispositif selon l'une des revendications 1 à 12 ou 20, dans lequel le dispositif de transfert (3) présente une plaque de support (110) commune pour la station de transfert (4), en particulier pour les deux stations de transfert (4), et la pince (36), plaque de support sur laquelle sont disposés la station de transfert (4) ou les stations de transfert (4) et la pince (36), cette dernière étant en particulier une plaque de support d'une seule pièce et en particulier une plaque de support en forme de L.

22. Dispositif selon l'une des revendications 1 à 11 ou 20 à 21, le dispositif de transfert (3) présentant, dans la zone de la pince (36), un capteur optique (120) qui peut être temporairement désactivé de manière contrôlée, lequel capteur est conçu et disposé pour détecter la présence ou l'absence de la boîte de Pétri dans la pince.

23. Dispositif selon l'une des revendications 1 à 11 ou 20 à 22, dans lequel un capteur (125) pour la présence ou l'absence du couvercle (49) dans la station de transfert est prévu à chaque station de transfert (3).

24. Dispositif selon l'une des revendications 1 à 11 ou 20 à 23, dans lequel la pince (36) est réalisée de telle sorte que sa position de base est la condition de préhension et en particulier dans lequel le au moins un élément de fixation (100. 101) est réalisé de telle sorte que sa position de base est la position ouverte, non fixe.

25. Dispositif selon l'une des revendications 1 à 11 ou 20 à 24, dans lequel la pince (36) présente au moins deux doigts (37), qui sont disposés respectivement sur un corps (38), les corps pouvant être rapprochés et éloignés l'un de l'autre de manière contrôlée et entraînée, et dans lequel chaque doigt (37) est fixé au corps associé de manière à pouvoir pivoter dans un plan horizontal, en particulier dans lequel chaque doigt est fixé à la tige en se centrant automatiquement dans une position de base non pivotée, et dans lequel en particulier chaque doigt (37) est fixé à la tige par sa face arrière (137) par l'intermédiaire d'un ressort à lames (138).

26. Dispositif selon l'une des revendications 1 à 11 ou 20 à 25, dans lequel les doigts (37) de la pince (36) sont conçus et disposés pour un contact essentiellement ponctuel de la boîte de Petri, en particulier pour un contact ponctuel au moyen des zones d'extrémité (140) des doigts.

27. Dispositif selon l'une des revendications 1 à 11 ou 20 à 26, dans lequel les doigts (37) de la pince (36) sont formés par une partie de base (145) et une partie d'échange (146) fixée de manière amovible à celle-ci, laquelle partie d'échange est conçue pour le contact ponctuel, et dans lequel en particulier la partie de base est formée d'un métal léger, en particulier de l'aluminium, et la partie d'échange est formée d'acier, en particulier d'acier à ressort.

28. Dispositif selon l'une des revendications 1 à 11 ou 20 à 28, dans lequel la pince (36) est conçue de telle sorte que la taille de l'angle W entre le centre Z de la zone de préhension et les parties de contact les plus extérieures, conçues et disposées pour le contact avec la boîte de Pétri, des doigts (37) de la pince (36) est de 80 à 120 degrés et de préférence de 85 à 100 degrés et de préférence d'environ 90 degrés.

29. Dispositif selon l'une des revendications 1 à 11 ou 20 à 28, dans lequel le dispositif de stockage (1) possède une deuxième porte auxiliaire (122) sur sa paroi (150), sur laquelle se trouve le dispositif de transfert (3), par laquelle porte auxiliaire l'approvisionnement ou l'enlèvement de boîtes de Pétri dans ou hors du dispositif de stockage peut être effectué au moyen du dispositif de manipulation (5) et d'un dispositif de transport (155) situé à l'extérieur du dispositif de stockage (1) sur la paroi citée de celui-ci, dans lequel la deuxième porte auxiliaire (122) en particulier se trouve dans le même axe vertical V que la première porte auxiliaire (22), en particulier en dessous de la première porte auxiliaire.

30. Dispositif selon l'une des revendications 1 à 11 ou 20 à 29, dans lequel le dispositif de transport (155) comprend deux dispositifs de levage (156, 157) superposés verticalement et entraînés de manière contrôlée, grâce auxquels des boîtes de Pétri peuvent être soulevées ou déposées indépendamment l'une de l'autre sur deux bandes transporteuses (158, 159) disposées l'une au-dessus de l'autre.

31. Dispositif selon l'une des revendications 1 à 11 ou 20 à 30, dans lequel le dispositif de levage comporte respectivement un capteur, au moyen duquel la présence ou l'absence d'une boîte de Pétri peut être établie.

32. Dispositif selon l'une des revendications 1 à 11 ou 20 à 31, dans lequel deux carrousels (12', 13') disposés séparément l'un de l'autre et l'un à côté de l'autre sont prévus dans le dispositif de stockage, qui peut être desservi par un dispositif de manipulation commun (5).

33. Appareil de laboratoire avec un dispositif selon l'une des revendications 1 à 11 ou 20 à 32, l'appareil de laboratoire présentant une commande et deux bandes transporteuses (158, 159) superposées et entraînées de manière contrôlée, les bandes transporteuses étant disposées et pouvant être commandées de telle sorte qu'elles sont conçues pour interagir avec les dispositifs de levage (156, 157) afin d'introduire des boîtes de Pétri dans le dispositif de stockage (1) de l'appareil ou de les en retirer.
